# EUROPEAN PATENT SPECIFICATION

(11) **EP 0 766 694 B1**
(45) Date of publication and mention of the grant of the patent: **05.08.1998**
(21) Application number: 95921816.5
(22) Date of filing: 02.06.1995
(51) Int. Cl.: C07K 5/02, A61K 38/05

(54) **PEPTIDE DERIVATIVES WITH METALLOPEPTIDASE INHIBITORY ACTIVITY**
PEPTIDDERIVATE MIT METALLOPEPTIDASEHEMMENDER AKTIVITÄT
DERIVES PEPTIDIQUES POSSEDANT UNE ACTIVITE D'INHIBITION DES METALLOPEPTIDASES

(30) Priority: 21.06.1994 IT MI941292
(43) Date of publication of application: 09.04.1997
(73) Proprietor: ZAMBON GROUP S.p.A., I-36100 Vicenza (IT)
(72) Inventor: PELLACINI, Franco, I-20151 Milano (IT); NORCINI, Gabriele, I-21010 Vizzola Ticino (IT); BOTTA, Daniela, I-22100 Como (IT); ROMAGNANO, Stefano, I-20090 Buccinasco (IT); SANTANGELO, Francesco, I-20148 Milano (IT)
(86) International application number: EP9502100
(87) International publication number: WO9535307

(56) References cited:
- EP-A- 0 126 974
- BIOORG. MED. CHEM. LETT. , vol. 4, no. 22, 1994 USA, pages 2715-20, DE LOMBAERT S ET AL 'Dual inhibition of neutral endopeptidase and angiotensin-converting enzyme by N-phosphonomethyl and N-carboxyalkyl dipeptides'
- J. MED. CHEM. , vol. 32, no. 4, 1989 WASHINGTON STATE UNIV.;COLL. PHARM.; PULLMAN; WA; USA (US), pages 898-903, HSIEH K H ET AL 'Topographic probes of angiotensin and receptor: potent angiotensin II agonist containing diphenylalanine and long-acting antagonists containing biphenylalanine and 2-indan amino acid in position 8'

## Description

The present invention relates to peptide derivatives with metallopeptidase inhibitory activity and, more particularly, it relates to carboxyalkylpeptide derivatives useful in the treatment of cardiovascular diseases as metallopeptidase inhibitors.

The pharmacologic interest towards the study of metallopeptidase inhibitory molecules derives from the role that said enzymes exert on the level of the cardiocirculatory system.

It is well-known, in fact, that compounds with angiotensin converting enzyme (ACE) inhibitory activity are mainly useful in the treatment of hypertension and of cardiac failure in that they inhibit the formation of angiotensin II, a substance which increases the blood pressure.

Compounds with endothelin converting enzyme (ECE) inhibitory activity are useful as anti-vasoconstrictors in that they inhibit the formation of endothelin, a 21 amino acid peptide with vasoconstrictor activity.

Instead, compounds with inhibitory activity of the neutral endopeptidase enzyme (NEP), also called enkephalinase, are useful as vasodilators in that the NEP enzyme is responsible for the inactivation, not only of endogenous enkephaline, but also of atrial natriuretic factor (ANF), a vasodilator hormone secreted by heart.

Therefore, even exerting their action on the cardiovascular system with different mechanisms of action, the compounds with metallopeptidase inhibitory activity are generally used, alone or in combination, in the treatment of hypertension, renal failure and congestive heart failure.

Within the field of the metallopeptidase inhibitors, the carboxyalkylpeptide derivatives are known since several years.

For instance, N-carboxyalkyl dipeptides with ACE-inhibitory activity are described in the U.S. patent No. 4,374,829 (Merck & Co., Inc.), in the U.S. patent No. 4,487,716 (American Home Products Corp.) and in the U.S. patent No. 4,666,906 (USV Pharmaceutical Corporation); N-carboxyalkyl dipeptides with enkephalinase-inhibitory activity are described in the U.S. patent No. 4,721,726 (Schering Corporation), in the European patent application No. 54862 (Schering Corporation), in the European patent application No. 38758 (Roques et al.) and in the U.S. patent No. 4,610,816 (Schering Corporation); N-carboxyalkylacylamino acids with ACE-inhibitory activity are described in the U.S. patent No. 4,105,789 (E.R. Squibb & Sons, Inc.).

None of the aforementioned documents describes carboxyalkylpeptide derivatives wherein the C-terminal amino acid derivative is a biphenylalanine derivative.

The International patent application No. 94/03481 (Schering Corporation) describes aminoalkyl derivatives of alpha,omega-diamino acids which are synthetic intermediates for the preparation of NEP and ACE inhibitors.

The European patent application No. 126974 (G.D.Searle & Co.) describes a considerable number of N-carboxyalkyl peptides, among which also the dipeptides of formula 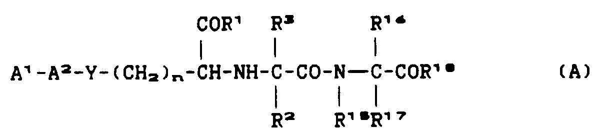 wherein the meanings of the R¹⁶ and R¹⁷ radicals, which represent the substituents of the C-terminal amino acid, are the following:
R¹⁶ represents hydrogen, alkyl or aralkyl;
R¹⁷ represents hydrogen, alkyl; substituted alkyl wherein the substituent can be one or more groups selected among hydroxy, alkoxy, aryloxy, aralkoxy, mercapto, alkylthio, arylthio, alkylsulphinyl, alkylsulphonyl, carboxy, carboxamido, carboxyalkyl, carboxyaralkyl, aralkoxycarbonylamino, amino, dialkylamino, acylamino, aroylamino and trihalomethyl groups; aralkyl, substituted aralkyl wherein the substituent in the aryl moiety can be one or more groups selected among halogen, alkyl, hydroxy, alkoxy, aralkoxy, amino, aminomethyl, cyano, alkylamino, dialkylamino, carboxy, sulphonamido, alkylthio, nitro and phenyl groups; or a heteroaralkyl;
and
- R¹⁸: represents amino, alkylamino, dialkylamino; substituted alkylamino wherein the substituent is amino, hydroxy, alkoxy, carboxy, carboxamido, carboxyalkyl, alkylthio, alkylsulphinyl or alkylsulphonyl; hydroxyamino, alkoxyamino, aralkylamino, alkoxy, aralkoxy or alkylaminoalkoxy groups.

The compounds of formula (A), among which are comprised but not exemplified the peptides wherein the C-terminal amino acid derivative is an ester or an amide of the biphenylalanine, are endowed with collagenase inhibitory activity and are thus useful in the treatment of articular diseases such as rheumatoid arthritis and correlated diseases.

As reported by the inventors in the same patent application (page 2 - last paragraph), the compounds of formula (A) are substantially devoid of ACE-inhibitory activity.

Now we have found carboxyalkylpeptide derivatives, wherein the C-terminal amino acid group is a biphenylalanine optionally substituted in the biphenyl moiety, endowed with inhibitory activity on the angiotensin converting enzyme as well as on the neutral endopeptidase enzyme (mixed ACE/NEP-inhibitory activity) which renders them particularly useful in the cardiovascular therapy.

Therefore, object of the present invention are the compounds of formula 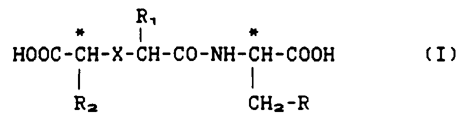 wherein
- R: is a biphenyl group optionally substituted by one or more substituents, the same or different, selected among halogen atoms, nitro groups, hydroxy groups, alkoxy, alkyl, thioalkyl or alkoxycarbonyl groups having from 1 to 6 carbon atoms in the alkyl moiety, C₁-C₃ alkyl groups containing one or more fluorine atoms, carboxy groups, amino or aminocarbonyl groups, acylamino groups, aminosulphonyl groups, mono- or di-alkylamino groups having from 1 to 6 carbon atoms in the alkyl moiety, mono- or di-alkylaminocarbonyl groups having from 1 to 6 carbon atoms in the alkyl moiety;
- R₁: is a hydrogen atom, a straight or branched C₁-C₆ alkyl or an arylalkyl group having from 1 to 6 carbon atoms in the alkyl moiety wherein the aryl is a phenyl, a biphenyl, a naphthyl or a 5 or 6 membered aromatic heterocycle with one or two heteroatoms selected among nitrogen, oxygen and sulphur, optionally substituted by one or more substituents, the same or different, selected among halogen atoms, hydroxy groups, alkoxy, alkyl, thioalkyl or alkoxycarbonyl groups having from 1 to 6 carbon atoms in the alkyl moiety, C₁-C₃ alkyl groups containing one or more fluorine atoms, carboxy groups, nitro groups, amino or aminocarbonyl groups, acylamino groups, aminosulphonyl groups, mono- or di-alkylamino groups having from 1 to 6 carbon atoms in the alkyl moiety, mono- or di-alkylaminocarbonyl groups having from 1 to 6 carbon atoms in the alkyl moiety;
- R₂: is a straight or branched C₁-C₆ alkyl optionally substituted by one or more substituents selected among amino, mono- or di-(C₁-C₆)alkylamino or aminocarbonyl groups, mono or di-(C₁-C₆)-alkylaminocarbonyl groups, an arylalkyl, an arylcarbonylaminoalkyl, an arylaminocarbonylalkyl or an arylalkylaminocarbonylalkyl group having from 1 to 6 carbon atoms in the alkyl moiety, the aryl being optionally substituted by one or more substituents, the same or different, selected among halogen atoms, hydroxy groups, alkoxy, alkyl, thioalkyl or alkoxycarbonyl groups having from 1 to 6 carbon atoms in the alkyl moiety, C₁-C₃ alkyl groups containing one or more fluorine atoms, carboxy groups, nitro groups, amino or aminocarbonyl groups, acylamino groups, aminosulphonyl groups, mono- or di-alkylamino groups having from 1 to 6 carbon atoms in the alkyl moiety, mono- or di-alkylaminocarbonyl groups having from 1 to 6 carbon atoms in the alkyl moiety;
- X: is a single bond or a -N(R₃)- group wherein R₃ is a hydrogen atom or a straight or branched C₁-C₄ alkyl group;
the carbon atoms marked with an asterisk are asymmetric carbon atoms;
and their pharmaceutically acceptable salts.

Object of the present invention are the compounds of formula I in the form of stereoisomeric mixture as well as in the form of single stereoisomers.

The compounds of formula I object of the present invention are endowed with a mixed ACE-inhibitory and NEP-inhibitory activity and are useful in the treatment of cardiovascular diseases such as hypertension, renal failure and congestive heart failure.

In the present description, unless otherwise specified, with the term biphenyl group we intend a 2-biphenyl, 3-biphenyl or 4-biphenyl group; with the term alkyl group we intend a straight or branched alkyl such as methyl, ethyl, n.propyl, isopropyl, n.butyl, sec-butyl, tert-butyl, isobutyl, n.pentyl, 2-pentyl, 3-pentyl, isopentyl, tert-pentyl, n.hexyl and isohexyl group; with the term halogen atom we intend a fluorine, chlorine, bromine or iodine atom; with the term acyl we intend an acyl group deriving from an aliphatic or aromatic carboxylic acid such as acetic, propionic, butyric and benzoic acid; with the term aryl we intend an aromatic group such as phenyl, 2-biphenyl, 3-biphenyl, 4-biphenyl, 1-naphthyl and 2-naphthyl or a 5- or 6-membered aromatic heterocyclic group containing 1 or 2 heteroatoms selected among nitrogen, oxygen and sulphur such as thiazole, isoxazole, oxazole, isothiazole, pyrazole, imidazole, thiophene, pyrrole, pyridine, pyrimidine and furan, optionally benzocondensed.

Examples of pharmaceutically acceptable salts of the compounds of formula I are the salts with alkali or alkali-earth metals and the salts with pharmaceutically acceptable organic bases.

Preferred compounds of formula I are the compounds wherein R is a 4-biphenyl group; R₁ is a straight or branched C₁-C₄ alkyl group or an arylalkyl having from 1 to 3 carbon atoms in the alkyl moiety wherein the aryl is a phenyl, a naphthyl, or a 5-membered aromatic heterocyclic group; R₂ is an arylalkyl, an arylalkylaminocarbonylalkyl or an arylaminocarbonylalkyl group having from 1 to 4 carbon atoms in the alkyl moiety wherein the aryl is a phenyl and X is a -N(R₃)- group wherein R₃ is a hydrogen atom.

Still more preferred compounds of formula I are the compounds wherein R is a 4-biphenyl group; R₁ is isopropyl, isobutyl or 2-thienylmethyl and R₂ is 2-phenylethyl, 2-phenylaminocarbonylethyl or benzylaminocarbonylmethyl.

Preferred examples of pharmaceutically acceptable salts of the compounds of formula I are the salts with alkali metals such as sodium, lithium and potassium.

Specific examples of preferred compounds of formula I, object of the present invention, are:
N-[N'-(1-carboxy-3-phenyl-propyl)-L-leucyl]-(1,1'-biphenyl-4-yl)-L-alanine;
N-[N'-(1-carboxy-3-phenyl-propyl)-L-valyl]-(1,1'-biphenyl-4-yl)-L-alanine;
N-[N'-(1-carboxy-3-phenyl-propyl)-(2-thienyl)-L-alanyl]-(1,1'-biphenyl-4-yl)-L-alanine;
N-[N'-(1-carboxy-3-phenylaminocarbonyl-propyl)-L-leucyl]-(1,1'-biphenyl-4-yl)-L-alanine;
N-[N'-(1-carboxy-3-phenylaminocarbonyl-propyl)-L-valyl]-(1,1'-biphenyl-4-yl)-L-alanine;
N-[N'-(1-carboxy-3-phenylaminocarbonyl-propyl)-(2-thienyl)-L-alanyl]-(1,1'-biphenyl-4-yl)-L-alanine;
N-[N'-(2-benzylaminocarbonyl-1-carboxy-ethyl)-L-leucyl]-(1,1'-biphenyl-4-yl)-L-alanine;
N-[N'-(2-benzylaminocarbonyl-1-carboxy-ethyl)-L-valyl]-(1,1'-biphenyl-4-yl)-L-alanine;
N-[N'-(2-benzylaminocarbonyl-1-carboxy-ethyl)-(2-thienyl)-L-alanyl]-(1,1'-biphenyl-4-yl)-L-alanine.

The preparation of the compounds of formula I, object of the present invention, comprises the reaction between a compound of formula 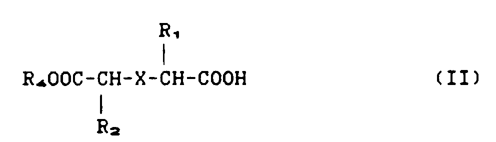 wherein R₁, R₂ and X have the above reported meanings and R₄ represents a protective group, preferably a C₁-C₄ alkyl, a phenyl or a phenylalkyl group having from 1 to 4 carbon atoms in the alkyl moiety;
and a biphenylalanine derivative of formula 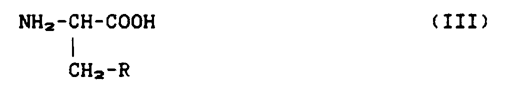 wherein R has the above reported meanings.

The condensation is carried out according to conventional techniques of the chemistry of peptides.

Before carrying out the reaction, it can be useful to properly protect the optional functional groups which could interfere in the reaction.

The optional protection is carried out according to conventional techniques.

For instance, it can be useful to protect the free carboxy function of the compound of formula III.

The evaluation of the usefulness of the optional protection as well as the selection of the kind of protection, with respect to the reaction to be carried out and to the functional groups to be protected, are within the normal knowledge of the man skilled in the art.

The removal of the optional protective groups is carried out according to conventional techniques.

For a general reference to the use of protective groups in organic chemistry see Theodora W. Greene and Peter G.M. Wuts "Protective Groups in Organic Synthesis", John Wiley & Sons, Inc.

The compounds of formula II and III are known or easily prepared according to known methods.

For instance, the compounds of formula II wherein X is a -N(R₃)-group can be prepared through the reaction of an α-keto ester of formula 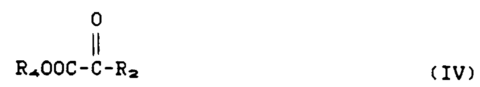 wherein R₂ and R₄ have the above reported meanings;
with an amino acid of formula 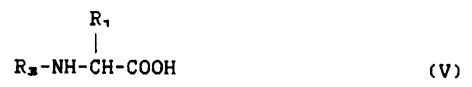 wherein R₁ and R₃ have the above reported meanings.

Alternatively, the compounds of formula II wherein X is a -N(R₃)-group can be prepared through the reaction of a α-hydroxy ester of formula 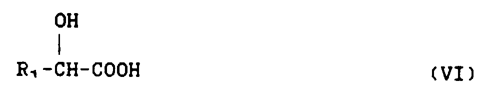 wherein R₁ has the above reported meanings;
with an amino acid of formula 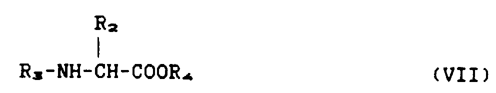 wherein R₂, R₃ and R₄ have the above reported meanings.

As previously pointed out, before carrying out the reaction it can be useful to properly protect the optional functional groups which could interfere in the reaction.

The compounds of formula II wherein X is a single bond, instead, are succinic acid derivatives and are preparable, for instance, according to the method described by Van Leeuwen et al. in Recl. Trav. Chim. Pays-Bas, **1980**, 99(3), 96-99.

A further process for the preparation of the compounds of formula I wherein X represents a -N(R₃)- group, object of the present invention, comprises the reaction of a compound of formula 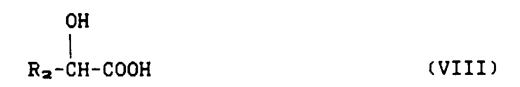 wherein R₂ has the above reported meanings;
with a dipeptide of formula 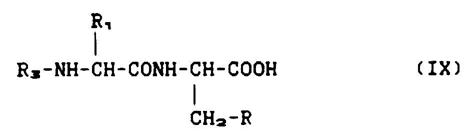 wherein R, R₁ and R₃ have the above reported meanings.

The dipeptides of formula IX, in their turn, can be easily prepared through a condensation reaction according to conventional techniques of the chemistry of peptides.

The compounds of formula I in the form of single stereoisomers are prepared by stereoselective synthesis or by separation of the stereoisomeric mixture according to conventional techniques.

Also the preparation of the salts of the compounds of formula I, object of the invention, is carried out according to conventional techniques.

The compounds of formula I object of the present invention are endowed with a mixed ACE-inhibitory and NEP-inhibitory activity and are useful in the treatment of cardiovascular diseases.

The inhibitory activity of the compounds of formula I has been evaluated by means of in vitro tests in comparison to known molecules with ACE-inhibitory or NEP-inhibitory activity (example 11).

Captopril, a drug known as the first oral active ACE-inhibitor (The Merck Index, XI ed. - No. 1773, pages 267-268), has been used as a reference compound for the ACE-inhibitory activity.

Thiorphan [DL-(3-mercapto-2-benzylpropanoyl)glycine], known molecule considered the parent compound of NEP-inhibitors and described for the first time by Roques et al. in Nature, Vol. 288, pages 286-288, (1980), has been used as a reference compound for the NEP-inhibitory activity.

The inhibitory activity of the compounds of formula I, expressed as IC₅₀ value, is pharmacologically significant in that it results in nM concentrations and is comparable to that of Captopril, as far as the ACE-inhibitory activity is concerned, and to that of thiorphan, as far as the NEP-inhibitory activity is concerned.

For a practical use in therapy, the compounds of formula I can be formulated in solid or liquid pharmaceutical compositions, suitable to oral or parenteral administration.

A further object of the present invention, therefore, are the pharmaceutical compositions containing a therapeutically effective amount of a compound of formula I in admixture with a carrier for pharmaceutical use.

Specific examples of pharmaceutical compositions according to the present invention are tablets, coated tablets, capsules, granulates, solutions and suspensions suitable to oral administration, solutions and suspensions suitable to parenteral administration.

The pharmaceutical compositions object of the present invention are prepared according to conventional techniques.

For a practical use in therapy it can be useful to formulate the compounds of formula I in the form of pro-drugs, namely, of derivatives which enable the in vivo release of the pharmacologically active molecule that is of the corresponding compounds of formula I. Specific examples of pro-drugs of the compounds of formula I are the esters of the carboxy group present in the carboxyalkyl function. Therefore, also the pro-drugs of the compounds of formula I are within the scope of the present invention.

The daily dose of the compound of formula I or of the corresponding pro-drug will depend on different factors such as the seriousness of the disease, the individual response of the patient or the kind of formulation but it is usually comprised between 0.1 mg and 50 mg per Kg of body weight divided into a single dose or into more daily doses.

With the aim of better illustrating the present invention the following examples are now given.

### Example 1

### Preparation of N-[(1S)-1-ethoxycarbonyl-3-phenyl-propyl]-L-leucine

A suspension of L-leucine (1 g; 7.62 mmoles) and 4-phenyl-2-oxo-butyric acid ethyl ester (1.89 g; 9.15 mmoles), prepared as described in Chem. Pharm. Bull., 32(5), 1800-1807 (1984), in absolute ethanol (40 ml), was hydrogenated into a Parr apparatus in the presence of palladium on charcoal at 10% (200 mg) at 40-50°C for 15 hours.

After having filtered off the catalyst and evaporated to dryness, the residue was purified by silica gel column chromatography (eluent CH₂Cl₂:CH₃OH=95:5).

The resultant solid was collected with petroleum ether and filtered. After drying under vacuum at 50°C, N-[(1S)-ethoxycarbonyl-3-phenyl-propyl]-L-leucine (150 mg) was thus obtained.
m.p. 103-105°C
¹H-NMR (200 MHz, CDCl₃): δ(ppm): 0.86-0.98 (m, 6H, CH₃-CH-CH₃); 1.26 (t, 3H, CH₂-CH₃); 1.40-2.12 (m, 5H, Ph-CH₂-CH₂, NH-CH-CH₂-CH); 2.57-2.82 (m, 2H, CH₂-Ph); 3.13-3.38 (m, 2H, CH-NH-CH); 4.18 (q, 2H, COOCH₂); 7.10-7.31 (m, 5H, aryl).

### Example 2

### Preparation of N-[N'-[(1S)-ethoxycarbonyl-3-phenyl-propyl]-L-leucyl]-(1,1'-biphenyl-4-yl)-L-alanine methyl ester

N,N'-dicyclohexylcarbodiimide (422 mg; 2.046 mmoles) was added, under stirring at room temperature, to a solution of N-[(1S)-1-ethoxycarbonyl-3-phenyl-propyl]-L-leucine (600 mg; 1.86 mmoles), prepared as described in example 1, and N-hydroxysuccinimide (214 mg; 1.86 mmoles) in dioxane (12 ml).

After 2 hours, dicyclohexylurea was filtered off and (1,1'-biphenyl-4-yl)-L-alanine methyl ester (493 mg; 1.69 mmoles) and triethylamine (0.282 ml; 2.03 mmoles) were respectively added to the resultant solution.

The reaction mixture was heated at 60°C for 3 hours.

After cooling and diluting with ethyl acetate, the reaction mixture was washed 3 times with water and dried on sodium sulphate.

By evaporating to dryness, a residue (1.5 g) which was purified by silica gel column chromatography (eluent 60-80°C petroleum ether:ethyl acetate=75:25) was obtained.

N-[N'-[(1S)-ethoxycarbonyl-3-phenyl-propyl]-L-leucyl]-(1,1'-biphenyl-4-yl)-L-alanine methyl ester (760 mg; 80% yield) was obtained as an oil.

¹H-NMR (200 MHz, CDCl₃): δ(ppm): 0.85-0.93 (m, 6H, CH₃-CH-CH₃); 1.22 (t, 3H, CH₂-CH₃); 1.28-1.98 (m, 5H, Ph-CH₂-CH₂, CH-CH₂-CH); 2.45--2.70 (m, 2H, CH₂-Ph); 3.00-3.08 (m, 1H, NH-CH); 3.12-3.19 (m, 2H, CH₂-biphenyl); 3.20-3.26 (m, 1H, CH-NH); 3.71 (s, 3H, COOCH₃); 4.11 (q, 2H, COOCH₂); 4.88-4.98 (m, 1H, CH-NHCO); 7.02-7.50 (m, 14H, aryl); 7.58 (d, 1H, CONH).

By working as described in examples 1 and 2 and by separating the diastereoisomeric mixtures by silica gel column chromatography, as indicated below, the following compounds were prepared:

### N-[N'-[(1S)-1-ethoxycarbonyl-4-phenyl-butyl]-L-leucyl]-(1,1'-biphenyl-4-yl)-L-alanine methyl ester

(eluent 40-60°C petroleum ether:ethyl acetate=70:30, Rf=0.38)
¹H-NMR (200 MHz, CDCl₃): δ(ppm): 0.85-0.92 (m, 6H, CH₃-CH-CH₃); 1.20-1.75 [m, 10H, COO-CH₂-CH₃, CH-CH₂-CH₂, CH₂-CH-CH(CH₃)₂]; 2.48-2.59 (m, 2H, CH₂-Ph); 3.00-3.25 (m, 4H, CH-NH-CH, CH₂-biphenyl); 3.70 (s, 3H, COO-CH₃); 4.07-4.19 (m, 2H, COO-CH₂); 4.85-4.95 (m, 1H, CH-NHCO); 7.05-7.60 (m, 15H, CONH, aryl).

### N-[N'-[(1R)-1-ethoxycarbonyl-4-phenyl-butyl]-L-leucyl]-(1,1'-biphenyl-4-yl)-L-alanine methyl ester

(eluent 40-60°C petroleum ether:ethyl acetate=70:30, Rf=0.31)
¹H-NMR (200 MHz, CDCl₃): δ(ppm): 0.80-0.89 (m, 6H, CH₃-CH-CH₃); 1.12-1.80 [m, 10H, COO-CH₂-CH₃, CH-CH₂-CH₂, CH₂-CH-CH(CH₃)₂); 2.52--2.62 (m, 2H, CH₂-Ph); 3.00-3.29 (m, 4H, CH-NH-CH, CH₂-biphenyl); 3.71 (s, 3H, COO-CH₃); 4.02-4.21 (m, 2H, COO-CH₂); 4.80-4.91 (m, 1H, CH-NHCO); 7.08-7.59 (m, 15H, CONH, aryl).

### N-[N'-[(1S)-1-ethoxycarbonyl-butyl]-L-leucyl]-(1.1'-biphenyl-4-yl)-L-alanine methyl ester

(eluent hexane:ethyl acetate=70:30, Rf=0.27)
¹H-NMR (200 MHz, CDCl₃): δ(ppm): 0.83-0.91 (m, 6H, CH₃-CH-CH₃); 1.11-1.75 [m, 10H, COO-CH₂-CH₃, CH-CH₂-CH₂, CH₂-CH-CH(CH₃)₂]; 2.95-3.19 (m, 4H, CH-NH-CH, CH₂-biphenyl); 3.72 (s, 3H, COO-CH₃); 4.05-4.19 (m, 2H, COO-CH₂); 4.85-4.98 (m, 1H, CH-NHCO); 7.13-7.60 (m, 9H, CONH, aryl).

### N-[N'-[(1R)-1-ethoxycarbonyl-butyl]-L-leucyl]-(1,1'-biphenyl-4-yl)-L-alanine methyl ester

(eluent hexane:ethyl acetate=70:30, Rf=0.23)
¹H-NMR (200 MHz, CDCl₃): δ(ppm): 0.82-0.91 (m, 6H, CH₃-CH-CH₃); 1.11-1.78 [m, 10H, COO-CH₂-CH₃, CH-CH₂-CH₂, CH₂-CH-CH(CH₃)₂]; 2.99--3.28 (m, 4H, CH-NH-CH, CH₂-biphenyl); 3.71 (s, 3H, COO-CH₃); 4.05-4.19 (m, 2H, COO-CH₂); 4.78-4.91 (m, 1H, CH-NHCO); 7.15-7.60 (m, 9H, CONH, aryl).

### Example 3

### Preparation of N-[N'-[(1S)-1-ethoxycarbonyl-3-phenyl-propyl]-L-leucyl]-(4'-methoxy-1,1'-biphenyl-4-yl)-L-alanine methyl ester

A solution of trifluoromethanesulphonic anhydride (1.26 ml; 7.68 mmoles) in methylene chloride (10 ml) was added dropwise to a solution of ethyl (R)-2-hydroxy-4-phenylbutyrate (1.6 g; 7.68 mmoles) and 1,8-bis(dimethylamino)naphthalene (1.64 g; 7.68 mmoles) in methylene chloride (16 ml) at -5°C while stirring.

After 30 minutes, a solution of N-(L-leucyl)-(4'-methoxy-1,1'-biphenyl-4-yl)-L-alanine methyl ester (2.55 g; 6.39 mmoles) and 1,8-bis(dimethylamino)naphthalene (1.36 g; 6.39 mmoles) in acetonitrile (25 ml) was therein added at -5°C while stirring.

The reaction mixture, kept at room temperature for 20 hours under stirring, was filtered and the resultant solution was evaporated to dryness at reduced pressure.

The residue was collected with ethyl acetate (25 ml) and brine (25 ml) and the phases were separated.

The organic phase was dried on sodium sulphate and evaporated.

The residue was silica gel column chromatographed (eluent ethyl acetate:petroleum ether=30:70) affording N-[N'-[(1S)-1-ethoxycarbonyl-3-phenyl-propyl]-L-leucyl]-(4'-methoxy-1,1'-biphenyl-4-yl)-L-alanine methyl ester (2.11 g) as a pale yellow solid.

¹H-NMR (200 MHz, CDCl₃): δ(ppm): 0.85-0.92 (m, 6H, CH₃-CH-CH₃); 1.20-2.00 [m, 8H, Ph-CH₂-CH₂-CH, CH₃-CH₂, CH₂-CH-CH(CH₃)₂]; 2.45-2.71 (m, 2H, CH₂-CH₂-Ph); 3.00-3.28 (m, 4H, CH-NH-CH, CH₂-biphenyl); 3.71 (s, 3H, COO-CH₃); 3.81 (s, 3H, biphenyl-OCH₃); 4.05-4.19 (m, 2H, COO-CH₂); 4.89-5.00 (m, 1H, CH-NHCO); 6.89-7.60 (m, 14H, CONH, aryl).

By working in a similar way 3 the following compounds were prepared:

### N-[N'-[(1S)-1-ethoxycarbonyl-3-phenyl-propyl]-L-valyl]-(1,1'-biphenyl-4-yl)-L-alanine methyl ester

¹H-NMR (200 MHz, CDCl₃): δ(ppm): 0.80-0.92 (m, 6H, CH₃-CH-CH₃); 1.21 (t, 3H, CH₃-CH₂); 2.40-2.70 (m, 2H, CH₂-Ph); 2.82-3.30 (m, 4H, CH-NH-CH-CH, CH₂-biphenyl); 3.72 (s, 3H, COO-CH₃); 4.05-4.19 (m, 2H, COOCH₂); 4.90-5.02 (m, 1H, CH-NHCO); 7.02-7.70 (m, 15H, CONH, aryl).

### N-[N'-[(1S)-1-ethoxycarbonyl-3-phenyl-propyl]-L-leucyl]-(3'-chloro-1,1'-biphenyl-4-yl)-L-alanine methyl ester

¹H-NMR (200 MHz, CDCl₃): δ(ppm): 0.85-0.95 (m, 6H, CH₃-CH-CH₃); 1.20-2.00 [m, 8H, Ph-CH₂-CH₂-CH, CH₃-CH₂, CH₂-CH-CH(CH₃)₂]; 2.40-2.70 (m, 2H, CH₂-CH₂-Ph); 3.00-3.30 (m, 4H, CH-NH-CH, CH₂-biphenyl); 3.71 (s, 3H, COOCH₃); 4.05-4.18 (m, 2H, COOCH₂); 4.89-5.00 (m, 1H, CH-NHCO); 7.01-7.61 (m, 14H, CONH, aryl).

### N-[N'-[(1S)-1-ethoxycarbonyl-3-phenyl-propyl]-(2-naphthyl)-L-alanyl]-(1.1'-biphenyl-4-yl)-L-alanine methyl ester

¹H-NMR (200 MHz, CDCl₃): δ(ppm): 1.05 (t, 3H, CH₃-CH₂); 1.60-1.90 (m, 2H, Ph-CH₂-CH₂-CH); 2.30-2.53 (m, 2H, CH₂-CH₂-Ph); 2.90-3.45 (m, 6H, CH-NH-CH, CH₂-biphenyl, CH₂-naphthyl); 3.69 (s, 3H, COOCH₃); 3.80-4.01 (m, 2H, COOCH₂); 4.89-5.01 (m, 1H, CH-NHCO); 6.90-7.85 (m, 22H, CONH, aryl).

### N-[N'-[(1S)-1-ethoxycarbonyl-3-phenyl-propyl]-(1-naphthyl)-L-alanyl]-(1,1'-biphenyl-4-yl)-L-alanine methyl ester

¹H-NMR (200 MHz, CDCl₃): δ(ppm): 0.80 (t, 3H, CH₃-CH₂); 1.60-1.88 (m, 2H, Ph-CH₂-CH₂-CH); 2.30-2.50 (m, 2H, CH₂-CH₂-Ph); 2.90-3.70 (m, 8H, COOCH₂, CH-NH-CH, CH₂-biphenyl, CH₂-naphthyl); 3.71 (s, 3H, COOCH₃); 4.88-5.00 (m, 1H, CH-NHCO); 6.91-8.20 (m, 22H, CONH, aryl).

### N-[N'-[(1S)-1-ethoxycarbonyl-3-phenyl-propyl]-(4-fluorophenyl)-L-alanyl]-(1,1'-biphenyl-4-yl)-L-alanine methyl ester

¹H-NMR (200 MHz, CDCl₃): δ(ppm): 1.15 (t, 3H, CH₃-CH₂); 1.60-1.90 (m, 2H, Ph-CH₂-CH₂-CH); 2.35-2.61 (m, 2H, CH₂-CH₂-Ph); 2.69-3.30 (m, 6H, CH-NH-CH, CH₂-biphenyl, CH₂-fluorophenyl); 3.70 (s, 3H, COOCH₃); 3.95-4.10 (m, 2H, COOCH₂); 4.88-4.90 (m, 1H, CH-NHCO); 6.95-7.60 (m, 19H, CONH, aryl).

### N-[N'-[(1S)-1-ethoxycarbonyl-3-phenyl-propyl]-(2-thienyl)-L-alanyl]-(1,1'-biphenyl-4-yl)-L-alanine methyl ester

¹H-NMR (200 MHz, CDCl₃): δ(ppm): 1.20 (t, 3H, CH₃-CH₂); 1.60-1.90 (m, 2H, Ph-CH₂-CH₂-CH); 2.35-2.61 (m, 2H, CH₂-CH₂-Ph); 2.98-3.31 (m, 6H, CH-NH-CH, CH₂-biphenyl, CH₂-thienyl); 3.70 (s, 3H, COOCH₃); 4.00-4.14 (m, 2H, COOCH₂); 4.91-5.01 (m, 1H, CH-NHCO); 6.90-7.70 (m, 18H, CONH, aryl).

### N-[N'-[(1S)-1-ethoxycarbonyl-3-phenyl-propyl]-L-phenylalanyl]-(1,1'-biphenyl-4-yl)-L-alanine methyl ester

¹H-NMR (200 MHz, CDCl₃): δ(ppm): 1.12 (t, 3H, CH₃-CH₂); 1.60-1.90 (m, 2H, Ph-CH₂-CH₂-CH); 2.31-2.51 (m, 2H, CH₂-CH₂-Ph); 2.71-3.35 (m, 6H, CH-NH-CH-CH₂-Ph, CH₂-biphenyl); 3.69 (s, 3H, COOCH₃); 3.90-4.10 (m, 2H, COOCH₂); 4.91-5.01 (m, 1H, CH-NHCO); 6.95-7.62 (m, 20H, CONH, aryl).

### N-[N'-[(1S)-1-methoxycarbonyl-2-methyl-propyl]-L-leucyl]-(1,1'-biphenyl-4-yl)-L-alanine methyl ester

¹H-NMR (200 MHz, CDCl₃): δ(ppm): 0.80-0.90 (m, 12H, 2CH₃-CH-CH₃); 1.20-1.88 [m, 4H, CH₂-CH-CH(CH₃)₂, NH-CH-CH] ; 2.91-3.15 (m, 4H, CH-NH-CH, CH₂-biphenyl); 3.63, 3.72 (2s, 6H, COOCH₃, OCH₃); 4.85-4.99 (m, 1H, CH-NHCO); 7.12-7.54 (m, 10H, CONH, aryl).

### N-[N'-[(1S)-1-methoxycarbonyl-3-methyl-butyl]-L-leucyl]-(1,1'-biphenyl-4-yl)-L-alanine methyl ester

¹H-NMR (200 MHz, CDCl₃): δ(ppm): 0.73-0.98 (m, 12H, 2CH₃-CH-CH₃); 1.10-1.80 [m, 6H, CH₂-CH-NH-CH-CH₂-CH-CH(CH₃)₂]; 2.95-3.25 (m, 4H, CH-NH-CH, CH₂-biphenyl); 3.65, 3.71 (2s, 6H, 2COOCH₃); 4.83-4.97 (m, 1H, CH-NHCO); 7.10-7.60 (m, 10H, CONH, aryl).

### N-[N'-[(1S)-1-benzyloxycarbonyl-2-phenyl-ethyl]-L-leucyl]-(1,1'-biphenyl-4-yl)-L-alanine benzyl ester

¹H-NMR (200 MHz, CDCl₃): δ(ppm): 0.75-0.83 (m, 6H, CH₃-CH-CH₃); 1.10-1.60 [m, 3H, CH₂-CH-CH(CH₃)₂]; 2.61-3.20 (m, 4H, CH₂-biphenyl, Ph-CH₂); 3.40-3.52 (m, 1H, CH-NH-CH-CO); 4.45-4.52 (m, 1H, Ph-CH₂-CH); 4.69-4.80 (m, 1H, CH-NHCO); 4.98-5.19 (m, 4H, 2Ph-CH₂-OCO); 6.95-7.51 (m, 2OH, CONH, aryl).

### N-[N'-[(1S)-3-benzylaminocarbonyl-1-methoxycarbonyl-propyl]-L-leucyl]-(1,1'-biphenyl-4-yl)-L-alanine methyl ester

¹H-NMR (200 MHz, CDCl₃): δ(ppm): 0.83-0.91 (m, 6H, CH₃-CH-CH₃) ; 1.30-2.10 [m, 6H, CH-NH-CH, CH₂-CH₂-CH, CH₂-CH-CH(CH₃)₂]; 2.21-2.30 (t, 2H, CO-CH₂); 2.95-3.14 (m, 4H, CH-NH-CH, CH₂-biphenyl); 3.61, 3.67 (2s, 6H, 2COOCH₃); 4.09-4.42 (m, 2H, Ph-CH₂-NH); 4.86-4.96 (m, 1H, CH-CH₂-biphenyl); 6.34 (bt, 1H, CO-NH-CH₂); 7.08-7.57 (m, 15H, CH-NH-CO, aryl).

### N-[N'-[(1S)-2-benzylaminocarbonyl-1-methoxycarbonyl-ethyl]-L-leucyl]-(1,1'-biphenyl-4-yl)-L-alanine methyl ester

¹H-NMR (200 MHz, CDCl₃): δ(ppm): 0.86 (d, 6H, CH₃-CH-CH₃); 1.15-1.76 [m, 3H, CH₂-CH(CH₃)₂]; 2.27 (bs, 1H, CH-NH-CH); 2.53 (d, 2H, CO-CH₂-CH); 2.95-3.60 (m, 4H, CH-NH-CH, CH₂-biphenyl); 3.69 (2s, 6H, 2COOCH₃); 4.21-4.44 (m, 2H, Ph-CH₂-NH); 4.80-4.92 (m, 1H, CH-CH₂-biphenyl); 6.26 (t, 1H, CO-NH-CH₂); 7.11-7.54 (m, 14H, aryl); 7.70 (d, 1H, CO-NH-CH).

### Example 4

### Preparation of N-[N'-[(1S)-1-methoxycarbonyl-3-phenylaminocarbonyl-propyl]-L-leucyl]-(1,1'-biphenyl-4-yl)-L-alanine methyl ester

A solution of trifluoromethanesulphonic anhydride (2.78 ml; 16.9 mmoles) in methylene chloride (10 ml) was added dropwise to a solution of benzyl (R)-2-hydroxy-4-methyl-valerate (3.76 g; 16.9 mmoles) and 1,8-bis(dimethylamino)naphthalene (3.6 g; 16.9 mmoles) in acetonitrile (37 ml) at -5°C while stirring.

After 30 minutes, a solution of (S)-5-anilide glutamic acid methyl ester (4 g; 16.9 mmoles) and 1,8-bis(dimethylamino)naphthalene (3 g; 13.5 mmoles) in acetonitrile (40 ml) was added dropwise at -5°C while stirring.

After 18 hours the reaction mixture was evaporated and the residue was collected with ethyl acetate (25 ml) and brine (25 ml).

The phases were separated and the organic phase was dried on sodium sulphate and evaporated to dryness obtaining a crude oil (7 g) which was purified by silica gel column chromatography (eluent ethyl acetate:petroleum ether=40:60).

The thus obtained oily compound (0.78 g) was dissolved in ethanol (30 ml) and hydrogenated into a Parr apparatus in the presence of palladium on charcoal at 10% (0.078 g).

After having filtered off the catalyst and evaporated to dryness, the resultant crude (0.57 g) was condensed at 0°C with (1,1'-biphenyl-4-yl)-L-alanine methyl ester hydrochloride (4.74 g; 1.62 mmoles) in tetrahydrofuran (12 ml), in the presence of a solution of hydroxybenzotriazole (0.22 g; 1.62 mmoles) and triethylamine (0.23 ml; 1.62 mmoles) in tetrahydrofuran (12 ml), by adding a solution of dicyclohexylcarbodiimide (0.335 g; 1.62 mmoles) in tetrahydrofuran (5 ml).

After 16 hours at room temperature, the reaction mixture was filtered and the resultant solution was evaporated.

The residue was collected with ethyl acetate (15 ml) and the solution was washed with 10% hydrochloric acid (2x10 ml), brine (2x10 ml), an aqueous solution of sodium bicarbonate at 5% (2x10 ml) and brine (10 ml), respectively.

By drying on sodium sulphate and evaporating to dryness it was obtained an oily residue (1 g) which, purified by silica gel column chromatography (eluent ethyl acetate:petroleum ether=60:40), furnished N-[N'-[(1S)-1-methoxycarbonyl-3-phenylaminocarbonyl-propyl]-L-leucyl]-(1,1'-biphenyl-4-yl)-L-alanine methyl ester (0.46 g).

¹H-NMR (200 MHz, CDCl₃): δ(ppm): 0.75-0.89 (m, 6H, CH₃-CH-CH₃); 1.10-2.05 [m, 5H, NHCO-CH₂-CH₂, CH₂-CH-CH(CH₃)₂]; 2.40-2.50 (m, 2H, NHCO-CH₂); 2.81-3.20 (m, 4H, CH-NH-CH, CH₂-biphenyl); 3.68, 3.81 (2s, 6H, 2COOCH₃); 4.98-5.10 (m, 1H, CH-NH-CO); 6.98-7.60 (m, 15H, CONH, aryl); 8.02 (s, 1H, Ph-NH).

By working in a similar way, the following compounds were prepared:

### N-[N'-[(1R)-1-methoxycarbonyl-3-phenylaminocarbonyl-propyl]-L-leucyl]-(1,1'-biphenyl-4-yl)-L-alanine methyl ester

¹H-NMR (200 MHz, CDCl₃): δ(ppm): 0.78-0.88 (m, 6H, CH₃-CH-CH₃); 1.20-2.20 [m, 5H, CH₂-CH-NH-CH-CH₂-CH-CH(CH₃)₂]; 2.31-2.41 (m, 2H, NHCO-CH₂-CH₂); 2.95-3.40 (m, 4H, CH-NH-CH, CH₂-biphenyl); 3.60, 3.72 (2s, 6H, 2COOCH₃); 4.82-4.98 (m, 1H, CH-NH-CO); 7.02-7.54 (m, 15H, CONH, aryl); 7.31 (s, 1H, Ph-NH).

### N-[N'-[(1S)-1-methoxycarbonyl-2-phenylaminocarbonyl-ethyl]-L-leucyl]-(1,1'-biphenyl-4-yl)-L-alanine methyl ester

¹H-NMR (200 MHz, CDCl₃): δ(ppm): 0.72-0.88 (m, 6H, CH₃-CH-CH₃; 1.20-1.80 [m, 3H, CH₂-CH-CH(CH₃)₂]; 2.60-3.22 (m, 5H, CH₂-CH-NH-CH, CH₂-biphenyl); 3.48-3.52 (m, 1H, CONH-CH₂-CH-NH) ; 3.71, 3.80 (2s, 6H, 2COOCH₃); 4.87-4.99 (m, 1H, CH-NHCO); 7.00-7.60 (m, 15H, CONH, aryl); 8.00 (s, 1H, Ph-NH).

### N-[N'-[(1S)-1-methoxycarbonyl-5-tert-butoxycarbonylamino-pentyl]-L-leucyl]-(1,1'-biphenyl-4-yl)-L-alanine methyl ester

¹H-NMR (200 MHz, CDCl₃): δ(ppm): 0.81-0.91 (m, 6H, CH₃-CH-CH₃); 1.20-1.70 [m, 18H, COO-C(CH₃)₃, CH₂-CH₂-CH₂-CH, CH₂-CH-CH(CH₃)₂]; 2.80-3.28 (m, 6H, OCO-NH-CH₂, CH-NH-CH, CH₂-biphenyl); 3.65, 3.75 (2s, 6H 2COOCH₃); 4.68 (bt, 1H, CO-NH-CH₂) ; 4.89-5.00 (m, 1H, CH-COO); 7.12-7.60 (m, 10H, NH-CO, aryl).

### Example 5

### Preparation of N-[N'-[(1S)-1-carboxy-3-phenyl-propyl]-L-leucyl]-(1.1'-biphenyl-4-yl)-L-alanine disodium salt (Compound 1)

A solution of sodium hydroxide 1N (5 ml) was added under stirring to a solution of N-[N'-[(1S)-1-ethoxycarbonyl-3-phenyl-propyl]-L-leucyl]-(1,1'-biphenyl-4-yl)-L-alanine methyl ester (700 mg; 1.25 mmoles), prepared as described in example 2, in ethanol (14 ml).

After one night, the solvent was evaporated under vacuum and the residue was collected with absolute ethanol (30 ml) and kept under stirring for 1 hour.

After 48 hours of rest, the precipitate was filtered washing with ethanol and ether.

By drying under vacuum at 40°C, N-[N'-[(1S)-1-carboxy-3-phenyl-propyl]-L-leucyl]-(1,1'-biphenyl-4-yl)-L-alanine disodium salt (570 mg; 81% yield) was obtained as a white solid.

¹H-NMR (200 MHz, D₂O): δ(ppm): 0.58-0.64 (m, 6H, CH₃-CH-CH₃); 1.04--1.11 (m, 2H, Ph-CH₂-CH₂); 1.18-1.55 (m, 3H, CH-CH₂-CH); 2.16-2.42 (m, 2H, CH₂-CH₂-Ph); 2.65-3.08 (m, 3H, CH-NH-CH, CH-CH₂-biphenyl); 4.32-4.38 (m, 1H, CH-COO); 6.92-7.44 (m, 14H, aryl).
Mass: 517 (M+H).

By working in a similar way the following compounds were prepared:

### N-[N'-[(1S)-1-carboxy-4-phenyl-butyl]-L-leucyl]-(1,1'-biphenyl-4-yl)-L-alanine dilithium salt (Compound 2)

¹H-NMR (200 MHz, D₂O): δ(ppm): 0.41-0.49 (m, 6H, CH₃-CH-CH₃); 0.91--1.44 (m, 7H, CH-CH₂-CH₂-CH₂, NH-CH-CH₂-CH) ; 2.16-2.36 (m, 2H, CH₂-CH₂-Ph) ; 2.82-2.73 (m, 2H, CH-NH-CH); 2.56-3.05 (m, 2H, CH-CH₂-biphenyl); 4.22-4.28 (m, 1H, CH-CH₂-Ph); 6.80-7.23 (m, 14H, aryl).
Mass: 531 (M+H)

### N-[N'-[(1R)-1-carboxy-4-phenyl-butyl]-L-leucyl]-(1,1'-biphenyl-4-yl)-L-alanine dilithium salt (Compound 3)

¹H-NMR (200 MHz, D₂O): δ(ppm): 0.49-0.54 (m, 6H, CH₃-CH-CH₃); 1.02--1.47 (m, 7H, CH-CH₂-CH₂-CH, NH-CH-CH₂-CH); 2.06-3.14 (m, 6H, Ph-CH₂-CH₂, CH-CH₂-biphenyl); 4.31-4.38 (m, 1H, CH-NH-CO); 6.92-7.42 (m, 14H, aryl).
Mass: 531 (M+H)

### N-[N'-[(1S)-1-carboxy-butyl]-L-leucyl]-(1,1'-biphenyl-4-yl)-L-alanine dilithium salt (Compound 4)

¹H-NMR (200 MHz, D₂O): δ(ppm): 0.54-0.65 (m, 9H, CH₃-CH₂, CH₃-CH-CH₃); 0.88-1.31 (m, 7H, CH₃-CH₂-CH₂, NH-CH-CH₂-CH); 2.66-3.12 (m, 4H, CH-NH-CH, CH₂-biphenyl); 4.32-4.38 (m, 1H, CH-CH₂-biphenyl); 7.10-7.47 (m, 9H, aryl).
Mass: 455 (M+H)

### N-[N'-[(1R)-1-carboxy-butyl]-L-leucyl]-(1,1'-biphenyl-4-yl)-L-alanine dilithium salt (Compound 5)

¹H-NMR (200 MHz, D₂O): δ(ppm): 0.58-0.66 (m, 9H, CH₃-CH₂, CH₃-CH-CH₃); 0.85-1.33 (m, 7H, CH₃-CH₂-CH₂, NH-CH-CH₂-CH); 2.75-3.17 (m, 4H, CH-NH-CH, CH₂-biphenyl); 4.36-4.43 (m, 1H, CH-CH₂-biphenyl); 7.19-7.57 (m, 9H, aryl).
Mass: 455 (M+H)

### N-[N'-[(1S)-1-carboxy-2-methyl-propyl]-L-leucyl]-(1,1'-biphenyl-4-yl)-L-alanine dilithium salt (Compound 6)

¹H-NMR (200 MHz, D₂O): δ(ppm): 0.58-0.69 (m, 12H, 2CH₃-CH-CH₃); 1.00-1.56 [m, 4H, CH-CH(CH₃)₂, CH₂-CH(CH₃)₂); 2.40 [d, 1H, NH-CH-CH(CH₃)₂]; 2.75-3.13 (m, 3H, CH₂-biphenyl, NH-CH-CONH); 4.34-4.41 (m, 1H, CH-CH-biphenyl); 7.17-7.56 (m, 9H, aryl).
Mass: 455 (M+H)

### N-[N'-[(1S)-1-carboxy-3-methyl-butyl]-L-leucyl]-(1,1'-biphenyl-4-yl)-L-alanine dilithium salt (Compound 7)

¹H-NMR (200 MHz, D₂O): δ(ppm): 0.55-0.70 (m, 12H, 2CH₃-CH-CH₃); 1.00-1.50 [m, 6H, 2CH₂-CH-CH(CH₃)₂]; 2.71-3.13 (m, 4H, CH-NH-CH, CH₂-biphenyl); 4.32-4.38 (m, 1H, CH-NHCO); 7.15-7.55 (m, 9H, aryl).
Mass: 469 (M+H)

### N-[N'-[(1R)-1-carboxy-3-phenylaminocarbonyl-propyl]-L-leucyl]-(1,1'--biphenyl-4-yl)-L-alanine dilithium salt (Compound 8)

¹H-NMR (200 MHz, D₂O): δ(ppm): 0.56-0.62 (m, 6H, CH₃-CH-CH₃); 1.08-1.28 [m, 3H, CH₂-CH-CH(CH₃)₂]; 1.46-1.57 (m, 2H, CO-CH₂-CH₂-CH) ; 1.79-2.15 (m, 2H, CO-CH₂); 2.67-3.15 (m, 4H, CH-NH-CH, CH₂-biphenyl); 4.45-5.38 (m, 1H, CH-COO); 6.97-7.43 (m, 14H, aryl).
Mass: 558 (M+H)

### Example 6

### Preparation of N-[N'-[(1S)-1-carboxy-3-phenyl-propyl]-L-leucyl]-(4'-methoxy-1,1'-biphenyl-4-yl)-L-alanine (Compound 9)

A solution of sodium hydroxide 1N (4.14 ml; 4.14 mmoles) was added dropwise, at 0-5°C and under stirring, to a solution of N-[N'-[(1S)--1-ethoxycarbonyl-3-phenyl-propyl)-L-leucyl]-(4'-methoxy-1,1'-biphenyl-4-yl)-L-alanine methyl ester (1.11 g; 1.88 mmoles), prepared as described in example 3, in ethanol (22 ml).

After one hour at 5°C, the reaction mixture was kept at room temperature for 20 hours and the solvent was evaporated at reduced pressure.

The residue was collected with water (10 ml) and acidified with an aqueous solution of hydrochloric acid at 10% (10 ml).

Tetrahydrofuran (10 ml) was therein added and the organic phase was separated, dried on sodium sulphate and evaporated to dryness.

The resultant crude was purified by silica gel column chromatography (eluent methylene chloride:methanol:acetic acid=90:10:1) affording N-[N'-[(1S)-1-carboxy-3-phenyl-propyl]-L-leucyl]-(4'-methoxy-1,1'-biphenyl-4-yl)-L-alanine (0.57 g).
m.p. 214-217°C

¹H-NMR (200 MHz, D₂O+NaOD): δ(ppm): 0.40-0.48 (m, 6H, CH₃-CH-CH₃); 0.89-1.60 (m, 5H, CH₂-CH-NH-CH-CH₂-CH); 2.12-2.38 (m, 2H, CH₂-CH₂-CH); 2.62-2.89 (m, 4H, CH-NH-CH, CH₂-biphenyl); 3.14 (s, 3H, OCH₃); 4.20-4.26 (m, 1H, CH-COO); 6.30-6.95 (m, 13H, aryl).
Mass: 547 (M+H)

By working in a similar way the following compounds were prepared:

### N-[N'-[(1S)-1-carboxy-3-phenyl-propyl]-L-valyl]-(1,1'-biphenyl-4-yl)-L-alanine (Compound 10)

m.p. 228-231°C

¹H-NMR (200 MHz, DMSO-d₆): δ(ppm): 0.79 (t, 6H, CH₃-CH-CH₃); 1.64-1.85 (m, 3H, CH₂-CH₂-CH, CH₃-CH-CH₃) ; 2.31-2.61 (m, 2H, CH₂-Ph); 2.73-3.18 (m, 4H, CH-NH-CH, CH₂-biphenyl); 4.53-4.64 (m, 1H, CH-COO); 7.00-7.58 (m, 14H, aryl); 7.95 (d, 1H, NH-CO).
Mass: 503 (M+H)

### N-[N'-[(1S)-1-carboxy-3-phenyl-propyl]-L-leucyl]-(3'-chloro-1,1'-biphenyl-4-yl)-L-alanine (Compound 11)

m.p. 214-216°C

¹H-NMR (200 MHz, DMSO-d₆): δ(ppm): 0.80 (t, 6H, CH₃-CH-CH₃); 1.20-1.29 [m, 2H, CH₂-CH(CH₃)₂]; 1.53-1.77 (m, 3H, CH₂-CH₂-Ph, CH₃-CH-CH₃); 2.32-2.63 (m, 2H, CH₂-Ph); 2.88-3.20 (m, 4H, CH-NH-CH, CH₂-biphenyl); 4.52-4.63 (m, 1H, CH-COO); 7.00-7.63 (m, 13H, aryl); 7.95 (d, 1H, NH-CO).
Mass: 551 (M+H)

### N-[N'-[(1S)-1-carboxy-3-phenyl-propyl]-(2-naphthyl)-L-alanyl]-(1,1'-biphenyl-4-yl)-L-alanine (Compound 12)

m.p. 217-219°C

¹H-NMR (200 MHz, DMSO-d₆): δ(ppm): 1.68-1.79 (m, 2H, CH₂-CH₂-Ph); 2.39-2.47 (m, 2H, CH₂-Ph); 2.87-3.11 (m, 5H, CH₂-biphenyl, CH-CH₂-naphthyl); 3.65-3.72 (m, 1H, CH-CH₂-CH₂); 4.55-4.62 (m, 1H, CO-NH-CH-CO); 6.95-7.85 (m, 21H, aryl).
Mass: 601 (M+H)

### N-[N'-[(1S)-1-carboxy-3-phenyl-propyl]-(1-naphthyl)-L-alanyl]-(1,1'-biphenyl-4-yl)-L-alanine (Compound 13)

m.p. 219-220°C

¹H-NMR (200 MHz, DMSO-d₆): δ(ppm): 1.62-1.75 (m, 2H, CH₂-CH₂-Ph); 2.34-2.62 (m, 2H, CH₂-Ph); 2.96-3.39 (m, 6H, CH₂-biphenyl, CH-NH-CH-CH₂-naphthyl); 4.50-4.60 (m, 1H, CH-COO); 7.02-8.09 (m, 22H, NH-CO, aryl).

### N-[N'-[(1S)-1-carboxy-3-phenyl-propyl]-(4-fluorophenyl)-L-alanyl]-(1,1'-biphenyl-4-yl)-L-alanine (Compound 14)

m.p. 229-231°C

¹H-NMR (200 MHz, DMSO-d₆): δ(ppm): 1.55-1.75 (m, 2H, CH₂-CH₂-Ph); 2.33-2.52 (m, 2H, CH₂-Ph); 2.68-2.86 (m, 2H, CH₂-fluorophenyl); 2.96-3.04 (m, 3H, CH-CH₂-CH₂-Ph, CH₂-biphenyl); 3.21-3.27 (m, 1H, CH-CH₂-Ph); 4.51-4.62 (m, 1H, CH-CH₂-biphenyl); 6.98-7.57 (m, 18H, aryl); 7.95 (d, 1H, NH-CO).
Mass: 569 (M+H)

### N-[N'-[(1S)-1-carboxy-3-phenyl-propyl]-(2-thienyl)-L-alanyl]-(1,1'-biphenyl-4-yl)-L-alanine (Compound 15)

m.p. 209-211°C

¹H-NMR (200 MHz, DMSO-d₆): δ(ppm): 1.58-1.78 (m, 2H, CH₂-CH₂-Ph); 2.36-2.51 (m, 2H, CH₂-Ph); 2.99-3.09 (m, 5H, CH-CH₂-CH₂, CH₂-biphenyl, CH₂-thienyl); 3.24-3.30 (m, 1H, CH-CH₂-thienyl); 4.49-4.59 (m, 1H, CH-CH₂-biphenyl); 6.84-7.57 (m, 17H, aryl); 8.00 (d, 1H, NH-CO).
Mass: 557 (M+H)

### N-[N'-[(1S)-1-carboxy-3-phenyl-propyl]-L-phenylalanyl]-(1,1'-biphenyl-4-yl)-L-alanine (Compound 16)

m.p. 221-222°C

¹H-NMR (200 MHz, DMSO-d₆): δ(ppm): 1.55-1.75 (m, 2H, CH₂-CH₂-Ph); 2.30-2.51 (m, 2H, CH₂-CH₂-Ph); 2.69-2.89 (m, 2H, CH-CH₂-Ph); 2.97--3.03 (m, 3H, CH-CH₂-CH₂, CH₂-biphenyl); 3.24-3.30 (m, 1H, CH-CH₂-Ph); 4.51-4.61 (m, 1H, CH-CH₂-biphenyl); 6.98-7.57 (m, 19H, aryl); 7.93 (d, 1H, NH-CO).
Mass: 551 (M+H)

### N-[N'-[(1S)-1-carboxy-5-tert-butoxycarbonylamino-pentyl]-L-leucyl]-(1,1'-biphenyl-4-yl)-L-alanine

¹H-NMR (200 MHz, DMSO-d₆): δ(ppm): 0.75-0.88 (m, 6H, CH₃-CH-CH₃); 1.10-1.70 [m, 18H, COO-C(CH₃)₃, CH₂-CH₂-CH₂-CH, CH₂-CH-CH(CH₃)₂]; 2.70-3.20 (m, 6H, OCO-NH-CH₂, CH₂-CH-CH₂, CH-CO-NH, CH₂-biphenyl); 4.45-4.60 (m, 1H, CH-COO); 6.70 (t, 1H, CO-NH-CH₂); 7.20-7.75 (m, 9H, aryl); 7.88 (d, 1H, CO-NH-CH).

### N-[N'-[(1S)-1-carboxy-3-phenylaminocarbonyl-propyl]-L-leucyl]-(1,1'-biphenyl-4-yl)-L-alanine (Compound 17)

¹H-NMR (200 MHz, DMSO-d₆): δ(ppm): 0.72-0.78 (m, 6H, CH₃-CH-CH₃); 1.09-1.17 (m, 2H, CH-CH₂-CH); 1.44-1.60 (m, 1H, CH₃-CH-CH₃); 1.69-1.98 (m, 2H, CO-CH₂-CH₂-CH); 2.30-2.54 (m, 2H, CO-CH₂); 2.84-3.21 (m, 4H, CH₂-biphenyl, CH-NH-CH); 4.30-4.39 (m, 1H, CH-COO); 6.93-7.63 (m, 14H, aryl); 7.85 (d, 1H, CO-NH-CH); 10.30 (s, 1H, NH-Ph).

### N-[N'-[(1S)-1-carboxy-2-phenylaminocarbonyl-ethyl]-L-leucyl]-(1,1'-biphenyl-4-yl)-L-alanine (Compound 18)

¹H-NMR (200 MHz, DMSO-d₆): δ(ppm): 0.84-0.89 (m, 6H, CH₃-CH-CH₃; 1.15-1.22 [m, 2H, CH₂-CH(CH₃)₂]; 1.51-1.72 (m, 1H, CH₃-CH-CH₃) ; 2.58-2.78 (m, 2H, CO-CH₂-CH-NH); 2.96-3.23 (m, 3H, CH-CO-NH, CH₂-biphenyl); 3.47-3.53 (m, 1H, CO-CH₂-CH-NH); 4.38-4.49 (m, 1H, CH-CH₂-biphenyl); 6.99-7.63 (m, 14H, aryl); 8.25 (d, 1H, CO-NH-CH); 10.12 (s, 1H, NH-Ph).
Mass: 546 (M+H)

### N-[N'-[(1S)-3-benzylaminocarbonyl-1-carboxy-propyl]-L-leucyl]-(1,1'-biphenyl-4-yl)-L-alanine (Compound 19)

¹H-NMR (200 MHz, DMSO-d₆): δ(ppm): 0.75-0.81 (m, 6H, CH₃-CH-CH₃); 1.12-1.22 (m, 2H, CH₂-CH₂-CH-NH); 1.46-1.87 [m, 3H, CH₂-CH(CH₃)₂]; 2.23-2.30 (m, 2H, NH-CO-CH₂); 2.83-3.18 (m, 4H, CH₂-biphenyl, CH-NH-CH); 4.01-4.35 (m, 3H, Ph-CH₂-NH, NH-CH-CH₂-biphenyl) ; 7.13-7.59 (m, 16H, 2COOH, aryl); 7.81 (bd, 1H, CO-NH-CH); 8.63 (bt, 1H, NH-CH₂).
Mass: 574 (M+H)

### N-[N'-[(1S)-2-benzylaminocarbonyl-1-carboxy-ethyl]-L-leucyl]-(1,1'-biphenyl-4-yl)-L-alanine (Compound 20)

¹H-NMR (200 MHz, DMSO-d₆): δ(ppm): 0.74-0.79 (m, 6H, CH₃-CH-CH₃); 1.15-1.22 [m, 2H, CH₂-CH(CH₃)₂]; 1.50-1.70 (m, 1H, CH₃-CH-CH₃); 2.42-2.65 (m, 2H, CO-CH₂-CH); 2.94-3.19 [m, 3H, CH-CH₂-CH(CH₃)₂, CH₂-biphenyl); 3.47-3.54 (m, 1H, CH-CH₂-CO); 4.19-4.43 (m, 3H, CH₂-NH, CH-CH₂-biphenyl); 7.22-7.60 (m, 14H, aryl); 8.18 (d, 1H, NH-CH-CH₂-biphenyl); 8.60 (t, 1H, NH-CH₂-Ph).
Mass: 558 (M-H)

### Example 7

### Preparation of N-[N'-[(1S)-5-amino-1-carboxy-pentyl]-L-leucyl]-(1,1'-biphenyl-4-yl)-L-alanine dihydrochloride (Compound 21)

A saturated solution of hydrochloric acid in ethyl ether (5 ml; 11% weight/volume) was added at room temperature and under stirring to a solution of N-[N'-[(1S)-1-carboxy-5-tert-butoxycarbonylamino-pentyl]-L-leucyl]-(1,1 '-biphenyl-4-yl)-L-alanine (1 g; 1.71 mmoles), prepared as described in example 6, in an ethyl ether:methylene chloride=1:1 mixture (30 ml).

After 16 hours the reaction mixture was evaporated and the residue was triturated with ethyl ether and filtered affording N-[N'-[(1S)-5-amino-1-carboxy-pentyl]-L-leucyl]-(1,1'-biphenyl-4-yl)-L-alanine dihydrochloride (0.83 g) as a white powder.

¹H-NMR (200 MHz, D₂O): δ(ppm): 0.69-0.74 (m, 6H, CH₃-CH-CH₃); 0.79--1.52 [m, 9H, NH-CH₂-CH(CH₃)₂, CH₂-CH(CH₃)₂]; 2.26-2.34 (m, 2H, CH₂-N); 2.64-3.32 (m, 2H, CH₂-biphenyl); 2.38-2.43, 2.64-3.71 (2m, 2H, CH-NH-CH); 4.72-4.80 (m, 1H, CH-CO); 7.18-7.61 (m, 9H, aryl).
Mass: 484 (M+H)

### Example 8

### Preparation of N-[N'-[(1S)-1-carboxy-2-phenyl-ethyl]-L-leucyl]-(1,1'-biphenyl-4-yl)-L-alanine disodium salt (Compound 22)

A solution of N-[N'-[(1S)-1-benzyloxycarbonyl-2-phenyl-ethyl]-L-leucyl]-(1,1'-biphenyl-4-yl)-L-alanine benzyl ester (3.5 g; 5.13 mmoles), prepared as described in example 3, in ethanol (114 ml) was hydrogenated into a Parr apparatus in the presence of palladium on charcoal at 10% (0.6 g).

After having filtered off the catalyst, the reaction mixture was evaporated to dryness and the resultant crude was purified by silica gel column chromatography (eluent methylene chloride:methanol:acetic acid=90:10:1).

The thus obtained compound (1.65 g) was treated with a solution of sodium hydroxide 1N (5.9 ml) at room temperature and the solution was evaporated to dryness.

The residue, triturated with acetonitrile and with ethanol and filtered, furnished N-[N'-[(1S)-1-carboxy-2-phenyl-ethyl]-L-leucyl]-(1,1'-biphenyl-4-yl)-L-alanine disodium salt (1.3 g) as a white powder.

¹H-NMR (200 MHz, D₂O): δ(ppm): 0.39 (d, 3H, CH₃-CH); 0.45 (d, 3H, CH₃-CH) ; 0.79-0.86 (m, 2H, CH-CH₂-CH); 0.97-1.16 (m, 1H, CH₃-CH-CH₃); 2.29-3.10 (m, 6H, CH-NH-CH-CH₂-Ph, CH₂-biphenyl); 4.12-4.19 (m, 1H, CH-COOH); 6.91-7.36 (m, 14H, aryl).
Mass: 503 (M+H)

### Example 9

### Preparation of N-(2,3-dimethyl-succinyl)-(1,1'-biphenyl-4-yl)-L-alanine methyl ester

Meso-2,3-dimethyl-succinic acid (1.5 g; 10.27 mmoles) was suspended in trifluoroacetic anhydride (15 ml) and kept under stirring up to complete dissolution (10 minutes).

The reaction mixture was stirred overnight at room temperature and the excess of anhydride was removed under reduced pressure.

The resultant oil was dissolved in methylene chloride (10 ml) and added dropwise to a solution of (1,1'-biphenyl-4-yl)-L-alanine methyl ester (3.6 g; 12.32 mmoles) in methylene chloride (20 ml) at 0°C and under stirring.

After 15 minutes the reaction mixture was kept at room temperature and the solution was stirred overnight.

The organic phase was washed with hydrochloric acid 1N (30 ml) and water (30 ml), dried on sodium sulphate and evaporated to dryness under reduced pressure.

The crude product was purified by flash chromatography [flash chromatography silica gel, (Baker - code 7024-00), eluent methylene chloride:methanol:acetic acid=90:10:1, pressure of nitrogen=0.2 atmospheres) furnishing N-(2,3-dimethyl-succinyl)-(1,1'-biphenyl-4-yl)-L-alanine methyl ester (3.4 g; 86% yield) as a mixture of two diastereoisomers.
m.p. 187-189°C

¹H-NMR (200 MHZ, CDCl₃): δ(ppm): 1.01-1.20 (m, 6H, 2CH₃-CH); 2.40-2.81 (m, 2H, CH-CH); 3.00-3.30 (m, 2H, CH₂-biphenyl); 3.75, 3.76 (2s, 3H, OCH₃); 4.87-5.00 (m, 1H, CH-COO); 6.30-6.40 (m, 1H, CONH); 7.10-7.60 (m, 9H, aryl).

### Example 10

### Preparation of N-(2,3-dimethyl-succinyl)-(1,1'-biphenyl-4-yl)-L-alanine (Compound 23)

Lithium hydroxide monohydrate (409 mg; 9.73 mmoles) was added to a suspension of N-(2,3-dimethyl-succinyl)-(1,1'-biphenyl-4-yl)-L-alanine methyl ester (1.5 g; 3.9 mmoles), prepared as described in example 9, in a tetrahydrofuran :water=2:1 mixture (15 ml).

The reaction mixture was kept under stirring at room temperature for one night.

After that, tetrahydrofuran was evaporated under reduced pressure and water (20 ml) was therein added.

The solution was acidified with hydrochloric acid 1N and the thus obtained precipitated was extracted with ethyl acetate (3x20 ml).

The organic phase was dried on sodium sulphate and evaporated at reduced pressure affording N-(2,3-dimethyl-succinyl)-(1,1'-biphenyl-4-yl)-L-alanine (1.2 g; 83% yield) as a mixture of two diastereoisomers.
m.p. 170-172°C

¹H-NMR (200 MHz, DMSO-d₆): δ(ppm): 0.61-0.99 (m, 6H, 2CH₃-CH); 2.14-2.45 (m, 2H, CH-CH); 2.79-3.18 (m, 2H, CH₂-biphenyl); 4.39-4.54 (m, 1H, CH-COO); 7.29-7.64 (m, 9H, aryl); 8.30-8.38 (m, 1H, CONH); 12.04 (bs, 2H, 2COOH).
Mass: 370 (M+H)

### Example 11

### "In vitro" evaluation of the pharmacologic activity

### a) NEP-inhibitory activity

The NEP-inhibitory activity was evaluated according to the method reported in the literature by C. Llorens et al., in Eur. J. Pharmacol., 69, (1981), 113-116.

Membranes from kidney cortex were prepared according to the following procedure.

By working at 0-4°C, kidneys were removed from male Sprague-Dawley rats weighing approximately 300 g.

Cortex was carefully dissected, finely minced and suspended in a homogenization buffer (10 mM sodium phosphate pH 7.4 containing 1 mM MgCl₂, 30 mM NaCl, 0.02% NaN₃) 1:15 weight/volume.

The tissue was then homogenized for 30 seconds using an Ultra-Turrax homogenizer.

Approximately 10 ml of homogenate were layered over 10 ml of sucrose (41% weight/volume) and centrifuged at 31200 rpm for 30 minutes at 4°C in a fixed angle rotor.

The membranes were collected from the buffer/sucrose interface, washed twice with 50 mM TRIS/HCl buffer (pH 7.4) and resuspended into the same buffer for storage.

The membranes were stored in small aliquots at -80°C until use. The NEP-inhibitory activity was evaluated by using the following method.

Aliquots of the membrane suspension prepared as above described (concentration 5 µg/ml of proteins) were preincubated in the presence of an aminopeptidase inhibitor (Bestatin - 1 mM) for 10 minutes at 30°C.

[³H][Leu⁵]-enkephaline (15 nM) and buffer TRIS/HCl pH 7.4 (50 mM) were added in order to obtain a final volume of 100 µl.

Incubation (20 minutes at 30°C) was stopped by adding HCl 0.1M (100 µl).

The formation of the metabolite [³H]Tyr-Gly-Gly was quantified by chromatography on polystyrene columns (Porapak Q).

The percentage of inhibition of the metabolite formation in the membrane preparations treated with the compounds of formula I and the reference compounds, in comparison to the untreated membrane preparations, was expressed as IC₅₀ value (nM).

### b) ACE-inhibitory activity

The ACE-inhibitory activity was evaluated according to the method reported in the literature by B. Holmquist et al., in Analytical Biochemistry 95, 540-548 (1979).

50 µM of ACE (250 mU/ml purified by lung rabbit, EC 3.4.15.1 SIGMA) with 50 µl of the compound of formula I or of the reference compounds, were preincubated in thermostated cuvettes at 37°C for 5 minutes.

The reaction was started by adding furylacryloylphenylalanylglycylglycine 0.8 mM (FAPGG-SIGMA).

Contemporaneously, by using a Beckman DU-50 spectrophotometer provided with a program for calculating delta A/minutes and regression coefficients of the enzyme kinetics curves, the absorbance at 340 nm was recorded in continuo for 5 minutes.

The percentage of the enzyme inhibition in the preparations treated with the compounds of formula I and with the reference compounds with respect to the untreated preparations was expressed as IC₅₀ value (nM).

The compounds of formula I were tested in the form of sodium salts.

We report in the following table 1 the ACE-inhibitory and NEP-inhibitory activity of compounds 1, 10, 15, 17 and 20 in comparison to thiorphan and Captopril.

**Table 1**

| ACE-inhibitory activity and NEP-inhibitory activity, expressed as IC₅₀ (nM), of compound 1, compound 10, compound 15, compound 17, compound 20, thiorphan and Captopril. | | |
|---|---|---|
| Compound | ACE-inhibitory Activity IC₅₀ (nM) | NEP-inhibitory activity IC₅₀ (nM) |
| 1 | 1.5 | 27.7 |
| 10 | 2 | 41 |
| 15 | 1.8 | 10 |
| 17 | 2.2 | 27 |
| 20 | 15 | 53 |
| thiorphan | 98.6 | 11.3 |
| Captopril | 2.8 | not active |

The compounds of formula I, object of the present invention, are endowed with a significant mixed ACE/NEP-inhibitory activity.

Moreover, the inhibitory activity of the compounds of formula I is comparable to the ACE-inhibitory activity of Captopril as well as to the NEP-inhibitory activity of thiorphan.

## Claims

1. A compound of formula 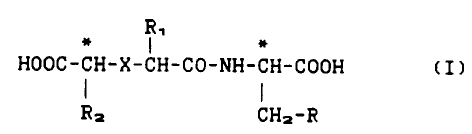 wherein
R is a biphenyl group optionally substituted by one or more substituents, the same or different, selected among halogen atoms, nitro groups, hydroxy groups, alkoxy, alkyl, thioalkyl or alkoxycarbonyl groups having from 1 to 6 carbon atoms in the alkyl moiety, C₁-C₃ alkyl groups containing one or more fluorine atoms, carboxy groups, amino or aminocarbonyl groups, acylamino groups, aminosulphonyl groups, mono- or di-alkylamino groups having from 1 to 6 carbon atoms in the alkyl moiety, mono- or di-alkylaminocarbonyl groups having from 1 to 6 carbon atoms in the alkyl moiety;
R₁ is a hydrogen atom, a straight or branched C₁-C₆ alkyl or an arylalkyl group having from 1 to 6 carbon atoms in the alkyl moiety wherein the aryl is a phenyl, a biphenyl, a naphthyl or a 5- or 6-membered aromatic heterocycle with one or two heteroatoms selected among nitrogen, oxygen and sulphur, optionally substituted by one or more substituents, the same or different, selected among halogen atoms, hydroxy groups, alkoxy, alkyl, thioalkyl or alkoxycarbonyl groups having from 1 to 6 carbon atoms in the alkyl moiety, C₁-C₃ alkyl groups containing one or more fluorine atoms, carboxy groups, nitro groups, amino or aminocarbonyl groups, acylamino groups, aminosulphonyl groups, mono- or di-alkylamino groups having from 1 to 6 carbon atoms in the alkyl moiety, mono- or di-alkylaminocarbonyl groups having from 1 to 6 carbon atoms in the alkyl moiety;
R₂ is a straight or branched C₁-C₆ alkyl optionally substituted by one or more substituents selected among amino, mono- or di-(C₁-C₆)alkylamino or aminocarbonyl groups, mono or di-(C₁-C₆)-alkylaminocarbonyl groups, an arylalkyl, an arylcarbonylaminoalkyl, an arylaminocarbonylalkyl or an arylalkylaminocarbonylalkyl group having from 1 to 6 carbon atoms in the alkyl moiety, the aryl being optionally substituted by one or more substituents, the same or different, selected among halogen atoms, hydroxy groups, alkoxy, alkyl, thioalkyl or alkoxycarbonyl groups having from 1 to 6 carbon atoms in the alkyl moiety, C₁-C₃ alkyl groups containing one or more fluorine atoms, carboxy groups, nitro groups, amino or aminocarbonyl groups, acylamino groups, aminosulphonyl groups, mono- or di-alkylamino groups having from 1 to 6 carbon atoms in the alkyl moiety, mono- or di-alkylaminocarbonyl groups having from 1 to 6 carbon atoms in the alkyl moiety;
X is a single bond or a -N(R₃)- group wherein R₃ is a hydrogen atom or a straight or branched C₁-C₄ alkyl group;
the carbon atoms marked with an asterisk are asymmetric carbon atoms;
and its pharmaceutically acceptable salts.

2. A compound according to claim 1 wherein R is a 4-biphenyl group; R₁ is a straight or branched C₁-C₄ alkyl group or an arylalkyl having from 1 to 3 carbon atoms in the alkyl moiety wherein the aryl is a phenyl, a naphthyl or a 5 membered aromatic heterocyclic group; R₂ is an arylalkyl, an arylalkylaminocarbonylalkyl or an arylaminocarbonylalkyl group having from 1 to 4 carbon atoms in the alkyl moiety wherein the aryl is a phenyl and X is a -N(R₃)- group wherein R₃ is a hydrogen atom.

3. A compound according to claim 1 wherein R is a 4-biphenyl group; R₁ is isopropyl, isobutyl or 2-thienylmethyl and R₂ is 2-phenylethyl, 2-phenylaminocarbonylethyl or benzylaminocarbonylmethyl.

4. A compound according to claim 1 in the form of a salt with an alkali metal selected among sodium, lithium and potassium.

5. A process for the preparation of a compound of formula I according to claim 1 which comprises the reaction between a compound of formula 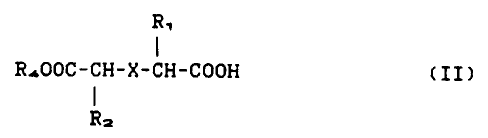 wherein R₁, R₂ and X have the meanings reported in claim 1 and R₄ represents a protective group selected among a C₁-C₄ alkyl, a phenyl or a phenylalkyl having from 1 to 4 carbon atoms in the alkyl moiety;
and a biphenylalanine derivative of formula 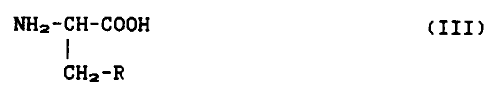 wherein R has the meanings reported in claim 1.

6. A process for the preparation of a compound of formula I according to claim 1 wherein X is a -N(R₃)- group which comprises the reaction between a compound of formula 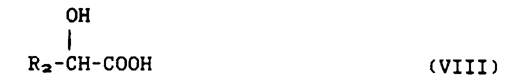 wherein R₂ has the meanings reported in claim 1;
and a dipeptide of formula 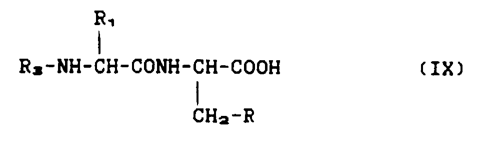 wherein R, R₁ and R₃ have the meanings reported in claim 1.

7. A pharmaceutical composition containing a therapeutically effective amount of a compound of formula I according to claim 1 in admixture with a carrier for pharmaceutical use.

8. A pharmaceutical composition according to claim 7 for the treatment of cardiovascular diseases.

9. N-[N'-(1-carboxy-3-phenyl-propyl)-L-leucyl]-(1,1'-biphenyl-4-yl)-L-alanine.

10. N-[N'-(1-carboxy-3-phenyl-propyl)-L-valyl]-(1,1'-biphenyl-4-yl)-L-alanine.

11. N-[N'-(1-carboxy-3-phenyl-propyl)-(2-thienyl)-L-alanyl]-(1,1'-biphenyl-4-yl)-L-alanine.

12. N-[N'-(1-carboxy-3-phenylaminocarbonyl-propyl)-L-leucyl]-(1,1'-biphenyl-4-yl)-L-alanine.

13. N-[N'-(1-carboxy-3-phenylaminocarbonyl-propyl)-L-valyl]-(1,1'-biphenyl-4-yl)-L-alanine.

14. N-[N'-(1-carboxy-3-phenylaminocarbonyl-propyl)-(2-thienyl)-L-alanyl]-(1,1'-biphenyl-4-yl)-L-alanine.

15. N-[N'-(2-benzylaminocarbonyl-1-carboxy-ethyl)-L-leucyl]-(1,1'-biphenyl-4-yl)-L-alanine.

16. N-[N'-(2-benzylaminocarbonyl-1-carboxy-ethyl)-L-valyl]-(1,1'-biphenyl-4-yl)-L-alanine.

17. N-[N'-(2-benzylaminocarbonyl-1-carboxy-ethyl)-(2-thienyl)-L-alanyl]-(1,1'-biphenyl-4-yl)-L-alanine.

18. A pharmaceutically acceptable salt of a compound according to anyone of claim 9 to 17.

## Patentansprüche

1. Verbindung der Formel 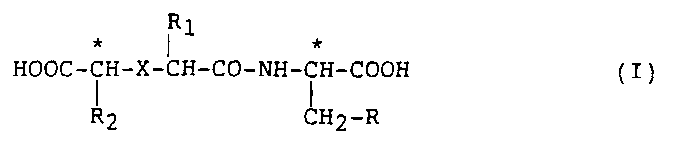 in welcher
R eine Biphenylgruppe ist, welche gegebenenfalls mit einem oder mehreren gleichen oder verschiedenen Substituenten substituiert ist, ausgewählt aus Halogenatomen, Nitrogruppen, Hydroxylgruppen, Alkoxy-, Alkyl-, Thioalkyl- oder Alkoxycarbonylgruppen mit 1 bis 6 Kohlenstoffatomen in der Alkylgruppierung, C₁-C₃-Alkylgruppen, welche ein oder mehrere Fluoratome enthalten, carboxylgruppen, Amino- oder Aminocarbonylgruppen, Acylaminogruppen, Aminosulfonylgruppen, Mono- oder Dialkylaminogruppen mit 1 bis 6 Kohlenstoffatomen in der Alkylgruppierung, Mono- oder Dialkylaminocarbonylgruppen mit 1 bis 6 Kohlenstoffatomen in der Alkylgruppierung;
R₁ ein Wasserstoffatom, ein lineares oder verzweigtes C₁-C₆-Alkyl oder eine Arylalkylgruppe mit 1 bis 6 Kohlenstoffatomen in der Alkylgruppierung ist, wobei das Aryl ein Phenyl, ein Biphenyl, ein Naphthyl oder ein 5- oder 6-gliedriger aromatischer Heterocyclus mit einem oder zwei aus Stickstoff, Sauerstoff und Schwefel ausgewählten Heteroatomen ist, gegebenenfalls substituiert ist mit einem oder mehreren gleichen oder verschiedenen Substituenten, ausgewählt aus Halogenatomen, Hydroxylgruppen, Alkoxy-, Alkyl-, Thioalkyl- oder Alkoxycarbonylgruppen mit 1 bis 6 Kohlenstoffatomen in der Alkylgruppierung, C₁-C₃-Alkylgruppen, welche ein oder mehrere Fluoratome enthalten, Carboxylgruppen, Nitrogruppen, Amino- oder Aminocarbonylgruppen, Acylaminogruppen, Aminosulfonylgruppen, Mono- oder Dialkylaminogruppen mit 1 bis 6 Kohlenstoffatomen in der Alkylgruppierung, Mono- oder Dialkylaminocarbonylgruppen mit 1 bis 6 Kohlenstoffatomen in der Alkylgruppierung;
R₂ ein lineares oder verzweigtes C₁-C₆-Alkyl, welches gegebenenfalls substituiert ist mit einem oder mehreren Substituenten, ausgewählt aus Amino, Mono- oder Di-(C₁-C₆)-alkylamino oder Aminocarbonylgruppen, Mono oder Di-(C₁-C₆)-alkylaminocarbonylgruppen, eine Arylalkyl-, eine Arylcarbonylaminoalkyl-, eine Arylaminocarbonylalkyl- oder eine Arylalkylaminocarbonylalkylgruppe mit 1 bis 6 Kohlenstoffatomen in der Alkylgruppierung ist, wobei das Aryl gegebenenfalls mit einem oder mehreren gleichen oder verschiedenen Substituenten substituiert ist, ausgewählt aus Halogenatomen, Hydroxylgruppen, Alkoxy-, Alkyl-, Thioalkyl- oder Alkoxycarbonylgruppen mit 1 bis 6 Kohlenstoffatomen in der Alkylgruppierung, C₁-C₃-Alkylgruppen, welche ein oder mehrere Fluoratome enthalten, Carboxylgruppen, Nitrogruppen, Amino- oder Aminocarbonylgruppen, Acylaminogruppen, Aminosulfonylgruppen, Mono- oder Dialkylaminogruppen mit 1 bis 6 Kohlenstoffatomen in der Alkylgruppierung, Mono- oder Dialkylaminocarbonylgruppen mit 1 bis 6 Kohlenstoffatomen in der Alkylgruppierung;
X eine Einzelbindung oder eine -N(R₃)-Gruppe ist, in welcher R₃ ein Wasserstoffatom oder eine lineare oder verzweigte C₁-C₄-Alkylgruppe ist,
wobei die mit einem Stern markierten Kohlenstoffatome asymmetrische Kohlenstoffatome sind;
und deren pharmazeutisch akzeptablen Salze.

2. Verbindung gemäß Anspruch 1, in welcher R eine 4-Biphenylgruppe ist; R₁ eine lineare oder verzweigte C₁-C₄-Alkylgruppe oder ein Arylalkyl mit 1 bis 3 Kohlenstoffatomen in der Alkylgruppierung ist, wobei das Aryl eine Phenyl-, eine Naphthyl- oder eine 5-gliedrige heterocyclische Gruppe ist; R₂ eine Arylalkyl-, eine Arylalkylaminocarbonylalkyl- oder eine Arylaminocarbonylalkylgruppe mit 1 bis 4 Kohlenstoffatomen in der Alkylgruppierung ist, wobei das Aryl ein Phenyl ist und X eine -N(R₃)-Gruppe ist, in welcher R₃ ein Wasserstoffatom ist.

3. Verbindung gemäß Anspruch 1, in welcher R eine 4-Biphenylgruppe ist; R₁ gleich Isopropyl, Isobutyl oder 2-Thienylmethyl ist und R₂ gleich 2-Phenylethyl, 2-Phenylaminocarbonylethyl oder Benzylaminocarbonylmethyl ist.

4. Verbindung gemäß Anspruch 1 in der Form eines Salzes mit einem Alkalimetall, ausgewählt aus Natrium, Lithium und Kalium.

5. Verfahren zur Herstellung einer Verbindung der Formel I gemäß Anspruch 1, umfassend die Reaktion zwischen einer Verbindung der Formel 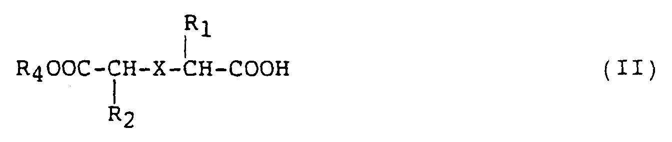 in welcher R₁, R₂ und X die in Anspruch 1 berichteten Bedeutungen besitzen und R₄ eine Schutzgruppe darstellt, ausgewählt aus einem C₁-C₄-Alkyl, einem Phenyl oder einem Phenylalkyl mit 1 bis 4 Kohlenstoffatomen in der Alkylgruppierung;
und einem Biphenylalaninderivat der Formel 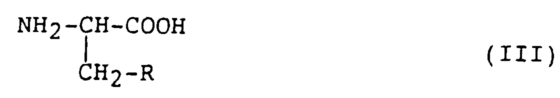 in welcher R die in Anspruch 1 berichteten Bedeutungen besitzt.

6. Verfahren zur Herstellung einer Verbindung der Formel I gemäß Anspruch 1, in welcher X eine -N(R₃)-Gruppe ist, umfassend die Reaktion zwischen einer Verbindung der Formel 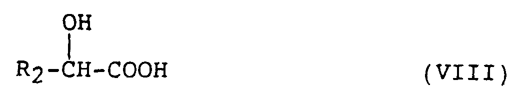 in welcher R₂ die in Anspruch 1 berichteten Bedeutungen besitzt;
und einem Dipeptid der Formel 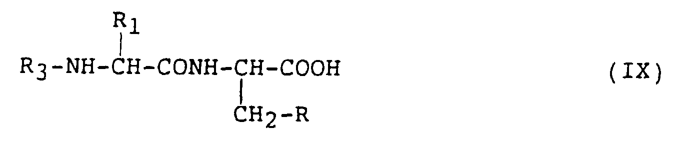 in welcher R, R₁ und R₃ die in Anspruch 1 berichteten Bedeutungen besitzen.

7. Pharmazeutische Zusammensetzung, welche eine therapeutisch wirksame Menge einer Verbindung der Formel I gemäß Anspruch 1 als Zusatz zu einem Träger für eine pharmazeutische Verwendung enthält.

8. Pharmazeutische Zusammensetzung gemäß Anspruch 7 für die Behandlung von Herz-Kreislauferkrankungen.

9. N-[N'-(1-Carboxy-3-phenylpropyl)-L-leucyl]-(1,1'-biphenyl-4-yl)-L-alanin.

10. N-[N'-(1-Carboxy-3-phenylpropyl)-L-valyl]-(1,1'-biphenyl-4-yl)-L-alanin.

11. N-[N'-(1-Carboxy-3-phenylpropyl)-(2-thienyl)-L-alanyl]-(1,1'-biphenyl-4-yl)-L-alanin.

12. N-[N'-(1-Carboxy-3-phenylaminocarbonylpropyl)-L-leucyl]-(1,1'-biphenyl-4-yl)-L-alanin.

13. N-[N'-(1-Carboxy-3-phenylaminocarbonylpropyl)-L-valyl]-(1,1'-biphenyl-4-yl)-L-alanin.

14. N-[N'-(1-Carboxy-3-phenylaminocarbonylpropyl)-(2-thienyl)-L-alanyl]-(1,1'-biphenyl-4-yl)-L-alanin.

15. N-[N'-(2-Benzylaminocarbonyl-1-carboxyethyl)-L-leucyl]-(1,1'-biphenyl-4-yl)-L-alanin.

16. N-[N'-(2-Benzylaminocarbonyl-1-carboxyethyl)-L-valyl]-(1,1'-biphenyl-4-yl)-L-alanin.

17. N-[N'-(2-Benzylaminocarbonyl-1-carboxyethyl)-(2-thienyl)-L-alanyl]-(1,1'-biphenyl-4-yl)-L-alanin.

18. Pharmazeutisch akzeptables Salz einer Verbindung gemäß einem der Ansprüche 9 bis 17.

## Revendications

1. Composé de formule : 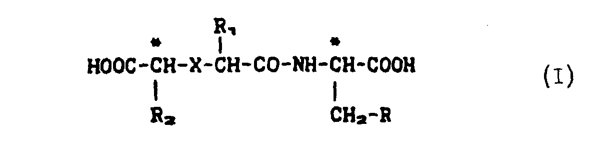 dans laquelle
R est un groupe biphényle éventuellement substitué par un ou plusieurs substituants, identiques ou différents, choisis parmi les atomes d'halogène, les groupes nitro, les groupes hydroxy, les groupes alcoxy, alkyle, thioalkyle ou alcoxycarbonyle ayant de 1 à 6 atomes de carbone dans la fraction alkyle, les groupes alkyle en C₁ à C₃ contenant un ou plusieurs atomes de fluor, les groupes carboxy, les groupes amino ou aminocarbonyle, les groupes acylamino, les groupes aminosulfonyle, les groupes mono- ou dialkylamino ayant de 1 à 6 atomes de carbone dans la fraction alkyle, les groupes mono- ou dialkylaminocarbonyle ayant de 1 à 6 atomes de carbone dans la fraction alkyle ;
R₁ est un atome d'hydrogène, un groupe alkyle en C₁ à C₆ linéaire ou ramifié ou un groupe arylalkyle ayant de 1 à 6 atomes de carbone dans la fraction alkyle où le groupe aryle est un groupe phényle, un groupe biphényle, un groupe naphtyle ou un hétérocycle aromatique à 5 ou 6 éléments avec un ou deux hétéroatomes choisis parmi l'azote, l'oxygène et le soufre, éventuellement substitué par un ou plusieurs substituants, identiques ou différents, choisis parmi les atomes d'halogène, les groupes hydroxy, les groupes alcoxy, alkyle, thioalkyle ou alcoxycarbonyle ayant de 1 à 6 atomes de carbone dans la fraction alkyle, les groupes alkyle en C₁ à C₃ contenant un ou plusieurs atomes de fluor, les groupes carboxy, les groupes nitro, les groupes amino ou aminocarbonyle, les groupes acylamino, les groupes aminosulfonyle, les groupes mono- ou dialkylamino ayant de 1 à 6 atomes de carbone dans la fraction alkyle, les groupes mono- ou dialkylaminocarbonyle ayant de 1 à 6 atomes de carbone dans la fraction alkyle ;
R₂ est un groupe alkyle en C₁ à C₆ linéaire ou ramifié éventuellement substitué par un ou plusieurs substituants choisis parmi les groupes amino, mono- ou dialkyl(en C₁ à C₆)amino ou aminocarbonyle, les groupes mono ou dialkyl(en C₁ à C₆)aminocarbonyle, un groupe arylalkyle, arylcarbonylaminoalkyl, arylaminocarbonylalkyle ou arylalkylaminocarbonylalkyle ayant de 1 à 6 atomes de carbone dans la fraction alkyle, le groupe aryle étant éventuellement substitué par un ou plusieurs substituants, identiques ou différents, choisis parmi les atomes d'halogène, les groupes hydroxy, alcoxy, alkyle, thioalkyle ou alcoxycarbonyle ayant de 1 à 6 atomes de carbone dans la fraction alkyle, les groupes alkyle en C₁ à C₃ contenant un ou plusieurs atomes de fluor, les groupes carboxy, les groupes nitro, les groupes amino ou aminocarbonyle, les groupes acylamino, les groupes aminosulfonyle, les groupes mono- ou dialkylamino ayant de 1 à 6 atomes de carbone dans la fraction alkyle, les groupes mono- ou dialkylaminocarbonyle ayant de 1 à 6 atomes de carbone dans la fraction alkyle ;
X est une liaison simple ou un groupe -N(R₃) dans lequel R₃ est un atome d'hydrogène ou un groupe alkyle en C₁ à C₄ linéaire ou ramifié ;
les atomes de carbone marqués d'une astérisque sont des atomes de carbone asymétriques ;
et ses sels acceptables sur le plan pharmaceutique.

2. Composé selon la revendication 1, dans lequel R est un groupe 4-biphényle ; R₁ est un groupe alkyle en C₁ à C₄ linéaire ou ramifié ou un groupe arylalkyle ayant de 1 à 3 atomes de carbone dans la fraction alkyle où le groupe aryle est un groupe phényle, un groupe naphtyle ou un groupe hétérocyclique aromatique à 5 éléments ; R₂ est un groupe arylalkyle, un groupe arylalkylaminocarbonylalkyle ou un groupe arylaminocarbonylalkyle ayant de 1 à 4 atomes de carbone dans la fraction alkyle où le groupe aryle est un groupe phényle et X est un groupe -N(R₃)- dans lequel R₃ est un atome d'hydrogène.

3. Composé selon la revendication 1, dans lequel R est un groupe 4-biphényle ; R₁ est un groupe isopropyle, isobutyle ou 2-thiénylméthyle et R₂ est un groupe 2-phényléthyle, 2-phénylaminocarbonyléthyle ou benzylaminocarbonylméthyle.

4. Composé selon la revendication 1, sous forme d'un sel avec un métal alcalin choisi parmi le sodium, le lithium et le potassium.

5. Procédé pour la préparation d'un composé de formule I selon la revendication 1, qui comprend la réaction entre un composé de formule 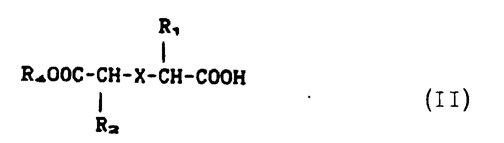 dans laquelle R₁, R₂ et X ont les significations rapportées dans la revendication 1 et R₄ représente un groupe protecteur choisi parmi un groupe alkyle en C₁ à C₄, un groupe phényle ou un groupe phénylalkyle ayant de 1 à 4 atomes de carbone dans la fraction alkyle;
et un dérivé biphénylalanine de formule : 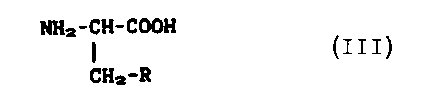 dans laquelle R possède les significations rapportées dans la revendication 1.

6. Procédé pour la préparation d'un composé de formule I, selon la revendication 1, dans lequel X est un groupe -N(R₃)- qui comprend la réaction entre un composé de formule : 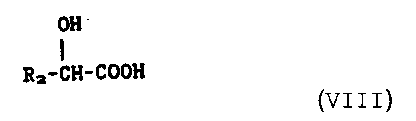 dans laquelle R₂ possède les significations rapportées dans la revendication 1;
et un dipeptide de formule : 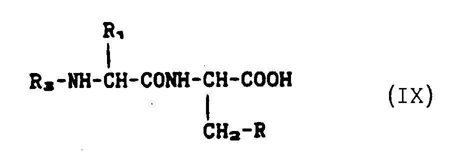 dans laquelle R, R₁ et R₃ ont les significations rapportées dans la revendication 1.

7. Composition pharmaceutique contenant une quantité efficace sur le plan thérapeutique d'un composé de formule I, selon la revendication 1 en mélange avec un véhicule pour une utilisation pharmaceutique.

8. Composition pharmaceutique selon la revendication 7, pour le traitement de maladies cardio-vasculaires.

9. N-[N'-(1-carboxy-3-phényl-propyl )-L-leucyl]-(1,1'-biphényl-4-yl )-L-alanine.

10. N-[N'-(1-carboxy-3-phényl-propyl)-L-valyl]-(1,1'-biphényl-4-yl)-L-alanine.

11. N-[N'-(1-carboxy-3-phényl-propyl)-(2-thiényl )-L-alanyl]-(1,1'-biphényl-4-yl)-L-alanine.

12. N-[N'-(1-carboxy-3-phénylaminocarbonyl-propyl)-L-leucyl]-(1,1'-biphényl-4-yl)-L-alanine.

13. N-[N'-(1-carboxy-3-phénylaminocarbonyl-propyl )-L-valyl]-(1,1'-biphényl-4-yl)-L-alanine.

14. N-[N'-(1-carboxy-3-phénylaminocarbonyl-propyl)-(2-thiényl)-L-alanyl]-(1,1'-biphényl-4-yl)-L-alanine.

15. N-[N'-(2-benzylaminocarbonyl-1-carboxy-éthyl)-L-leucyl]-(1,1'-biphényl-4-yl)-L-alanine.

16. N-[N'-(2-benzylaminocarbonyl-1-carboxy-éthyl)-L-valyl]-(1,1'-biphényl-4-yl)-L-alanine.

17. N-[N'-(2-benzylaminocarbonyl-1-carboxy-éthyl)-(2-thiényl)-L-alanyl]-(1,1'-biphényl-4-yl)-L-alanine.

18. Sel acceptable sur le plan pharmaceutique d'un composé selon l'une quelconque des revendications 9 à 17.
